# EUROPÄISCHE PATENTSCHRIFT

(11) **EP 2 350 295 B1**
(45) Veröffentlichungstag und Bekanntmachung des Hinweises auf die Patenterteilung: **15.05.2013**
(21) Anmeldenummer: 09748225.1
(22) Anmeldetag: 08.10.2009
(51) Int. Cl.: C12N 15/86, A61K 35/76, C12N 5/10, C12N 5/074

(54) **LCMV-GP-VSV-PSEUDOTYPVEKTOREN UND TUMORINFILTRIERENDE VIRENPRODUZENTENZELLEN ZUR THERAPIE VON TUMOREN**
LCMV-GP-VSV-PSEUDOTYPED VECTORS AND TUMOR-INFILTRATING VIRUS-PRODUCING CELLS FOR THE THERAPY OF TUMORS
VECTEURS PSEUDOTYPÉS LCMV-GP-VSV ET CELLULES HÔTES DE PRODUCTION DE VIRUS POUR INFILTRATION DANS LES TUMEURS AUX FINS DE TRAITEMENT DES TUMEURS

(30) Priorität: 08.10.2008 DE 102008050860
(43) Veröffentlichungstag der Anmeldung: 03.08.2011
(60) Teilanmeldung: 13153949.6
(73) Patentinhaber: Von Laer, Dorothee, 6020 Innsbruck (AT)
(72) Erfinder: VON LAER, Dorothee, 6020 Innsbruck (AT); HEIMANN, Tsanan, 55124 Mainz (DE)
(74) Vertreter: Meier, Jürgen
(86) Internationale Anmeldenummer: PCT/EP2009/007230
(87) Internationale Veröffentlichungsnummer: WO 2010/040526

(56) Entgegenhaltungen:
- WO-A1-2006/008074
- WO-A2-03/005964
- FISCHER YVONNE HEIDEMARIE ET AL: "A retroviral packaging cell line for pseudotype vectors based on glioma-infiltrating progenitor cells." THE JOURNAL OF GENE MEDICINE, Bd. 9, Nr. 5, Mai 2007 (2007-05), Seiten 335-344, XP009129737 ISSN: 1099-498X
- IRIE TAKASHI ET AL: "Modifications of the PSAP region of the matrix protein lead to attenuation of vesicular stomatitis virus in vitro and in vivo." THE JOURNAL OF GENERAL VIROLOGY, Bd. 88, Nr. Pt 9, September 2007 (2007-09) , Seiten 2559-2567, XP002569284 ISSN: 0022-1317
- AHMED MARYAM ET AL: "Immune response in the absence of neurovirulence in mice infected with m protein mutant vesicular stomatitis virus." JOURNAL OF VIROLOGY SEP 2008, Bd. 82, Nr. 18, September 2008 (2008-09), Seiten 9273-9277, XP002569285 ISSN: 1098-5514

## Beschreibung

Die vorliegende Erfindung betrifft vom Virus der vesikulären Stomatitis (VSV) abgeleitete rekombinante Viren und virale Vektoren, die ein für das Glykoprotein GP des Lymphozytären Choriomeningitisvirus (LCMV) kodierendes Gen enthalten, Verpackungszellen, die LCMV-GP-pseudotypisierte VSV-Virionen produzieren sowie die Verwendung der genannten Virionen und Verpackungszellen zur Herstellung einer pharmazeutischen Zusammensetzung zur Therapie solider Tumoren.

### BESCHREIBUNG DES STANDES DER TECHNIK

Maligne Gliome, die größte Gruppe primär intrakranieller Hirntumoren, stellen ein bisher ungelöstes therapeutisches Problem dar. Obwohl durch intensive Grundlagenforschung das Verständnis der Biologie dieser Tumoren gewachsen ist, sind klinischer Verlauf und Prognose weiterhin sehr schlecht.

Maligne Gliome sind Tumore neuroepithelialen Ursprungs und werden zytologisch in Ependymome, Oligodendrogliome, Oligo-Astrozytome, Astrozytome und Glioblastome unterteilt. Diffus infiltrierende Astrozytome (WHO Grad II-IV) bilden mit einem Anteil von über 60% die größte Gruppe intrakranieller Tumore. Als Gradierungsschema für Astrozytome hat sich die 2001 revidierte WHO-Klassifikation weitestgehend durchgesetzt. Nach der WHO werden hirneigene Tumoren mittels histologischer Kriterien 4 Malignitätsgraden zugeordnet (Kleihues and Cavenee, 2000). Die Prognose diffus infiltrierender Astrozytome ist im allgemeinen schlecht. Sie ist zum einen abhängig von dem Malignitätsgrad, zum anderen von der Lokalisation des Tumors und dem Therapieverfahren. Mittlere Überlebenszeiten sind für Patienten mit einem Astrozytom WHO-Grad II mehr als 5 Jahre, mit einem Astrozytom WHO-Grad III 2 bis 5 Jahre und mit einem Astrozytom-Glioblastom des WHO-Grades IV (=Glioblastom) weniger als 1 Jahr.

Die molekulare Pathogenese von Tumoren ist ein komplexer Prozess und beruht auf Mutationen von verschiedenen Genen, die für die Steuerung des Zellzyklus verantwortlich sind. Mutationen im Tumor-Suppressorgen p53 sind die häufigsten nachgewiesenen Veränderungen bei humanen Tumoren und sind mitverantwortlich sowohl für die Entstehung von niedriggradigen Astrozytomen als auch für die Progression zum sekundären Glioblastom. Primär entstandene Glioblastome weisen dagegen nur sehr selten p53-Mutationen auf. Ein weiteres Gen, das eine Malignisierungstendenz von diffusen Astrozytomen anzeigt, wird auf dem langen Arm von Chromosom 19 vermutet. Weitere bei Glioblastomen häufig veränderte Gene sind die Onkogene MDM2 und MDM4 sowie das Tumorsuppressorgen p14ARF, die an der p53-abhängigen Kontrolle des Zellzyklus involviert sind (Dai and Holland, 2001). Eine Amplifikation des EGF-Rezeptor-Gens wird bei 30-40% der primären Glioblastome beobachtet und ist somit das am häufigsten amplifizierte Onkogen bei dieser Tumorgruppe (Holland, 2001). Der überwiegende Anteil maligner Gliome spricht schlecht auf eine Chemo- oder Radiotherapie an. Der Grund hierfür wird in Mutationen von Zellzyklus-assoziierten Genen vermutet, die auch an der Regulierung der Apoptose beteiligt sind.

Effektivere Therapieverfahren für maligne Gliome sind dringend erforderlich, da von den bestehenden Therapieverfahren, wie der Chemo- oder Strahlentherapie, keine wesentlichen Verbesserungen für die Prognose der Erkrankung mehr erwartet werden. Die Gentherapie des Glioblastoms bietet dagegen vielversprechende Möglichkeiten, die es zu nutzen gilt. Eine Vielzahl verschiedener, sehr wirksamer Gene wurde zu diesem Zweck entwickelt. Zu den meisten liegen auch tierexperimentelle Daten vor (Shir und Levitzki, 2001). Diese therapeutischen Gene lassen sich vier verschiedenen Wirkprinzipien zuordnen:
(i) Das Genprodukt von so genannten Suizidgenen wandelt Vorläufer-Substanzen in zytotoxische Moleküle um. Ein Beispiel ist die Thymidinkinase (TK) des Herpes Simples Virus (HSV) in Verbindung mit der Gabe von Ganciclovir. Von besonderem Vorteil ist, dass das toxische Ganciclovirtriphosphat in benachbarte Zellen diffundiert, wodurch es zu einem "bystander" Effekt kommt. Die Aktivität dieses Enzyms konnte in den letzten Jahren noch weiter erhöht werden. HSV TK ist derzeit eine sehr effiziente Möglichkeit Tumorzellen, aber auch implantierte Vektorproduzentenzellen, zu eliminieren.
(ii) Die Expression immunstimulierender Zytokine, wie dem IL-4, im Tumor kann die natürliche Abwehr gegen die Tumorzellen stimulieren.
(iii) Die Sekretion von anti-angiogenetischen Proteinen, wie dem Endostatin, führt zu einem Mangel an Blutgefäßen und damit der Nährstoffversorgung in dem sehr stoffwechselaktiven Tumorgewebe. Der Tumor wird quasi "ausgehungert".
(iv) Schließlich wurden eine Reihe von Genen beschrieben, die in die Signaltransduktion oder den Zellzyklus der Tumorzelle eingreifen, um das ungehemmte Wachstum dieser Zellen zu inhibieren. Allerdings sind die Anwendungsmöglichkeiten dieser Gene in der Klinik begrenzt, da diese Gene nur in der genmodifizierten Zelle selbst wirken und keinen "bystander" Effekt wie die zuerst genannten Wirkprinzipien ausüben. Dies bedeutet, dass um einen therapeutischen Effekt zu erzielen, praktisch alle malignen Zellen genetisch modifiziert werden müssten, was auch mit idealen Vektorsystemen aussichtslos ist.

Durch die Vielzahl der vorhandenen, sehr effektiven Wirkprinzipien ist die wichtigste Voraussetzung für eine erfolgreiche Gentherapie des Glioblastoms geschaffen. Ein bisher nicht gelöstes Problem stellt allerdings der bislang ineffiziente Gentransfer und eine mangelnde Expression des therapeutischen Gens in den Zielzellen dar. Dies ist auch der Grund warum, trotz der Vielzahl effizienter therapeutischer Gene, die Gentherapie des Glioblastoms in der Klinik scheiterte.

Der Vorteil des viralen Gentransfers gegenüber physikochemischen Transfektionsverfahren besteht in der höheren Gentransferrate und der längerfristigen Expression der Gene, da die Viren besonders effiziente Mechanismen entwickelt haben, um ihr Genom in Zellen einzuführen und zu exprimieren. Als onkolytische Viren (OV) werden derzeit vor allem replikationskompetente Viren eingesetzt, unter anderem Herpes-Simplex Virus (HSV), Adenoviren (Ad), Newcastle Disease Virus (NDV), Reoviren und das Virus der vesikulären Stomatitis (VSV). Für eine optimale Virotherapie des Glioblastoms sollten OV folgende Merkmale aufweisen:
(i) sie sollten einen tumorspezifischen Tropismus haben, wodurch die Virusreplikation und Zelllyse auf das Tumorgewebe beschränkt bleibt. Diese Eigenschaft kann durch Modifikation der viralen Hülle oder durch die Verwendung von tumorspezifischen Promotoren verstärkt werden. Da man davon ausgeht, dass sich nur ein kleiner Teil der Gliomzellen während der Behandlung teilen, sind Viren, die sowohl ruhende als auch proliferierende Zellen infizieren, von Vorteil.
(ii) Aus sicherheitsrelevanten Gesichtspunkten heraus sind für die klinische Anwendung Viren mit hoher genetischer Stabilität und einer geringen Toxizität außerhalb des Tumorgewebes besonders geeignet. Dies ermöglicht hohe Virustiter und eine Aufreinigung der Vektoren unter GMP-Bedingungen. Idealerweise sollte das OV für den Menschen apathogen sein und eine geringe Durchseuchungsrate in der Bevölkerung aufweisen. Eine bereits vorhandene Immunität würde zur vorzeitigen Neutralisation des Virus führen und damit eine effiziente Therapie verhindern.

Prominente Beispiele für onkolytische Viren in der Therapie des Glioblastoms sind die attenuierten HSV Varianten G207 und 1716 und das Adenovirus ONYX-015. Ein ernstzunehmender Einwand für die Verwendung von onkolytischem HSV zur Behandlung von ZNS Tumoren ist seine starke Replikation in normalen Gehirnzellen, wodurch es zu einer lebensbedrohlichen Enzephalitis kommen kann. Zudem besteht neben einer potentiellen Persistenz, die Möglichkeit einer Reaktivierung von latentem HSV. Durch Deletion mehrerer Gene wurde die HSV Variante 1716 generiert, die selektiv in schnell proliferierenden Zellen des ZNS, aber nicht in postmitotischen Neuronen repliziert. Dadurch konnte die Neurotoxizität von HSV signifikant reduziert werden. Die Behandlung von experimentellen Gliomen in der Ratte und in der Maus mit HSV 1716 führte zur selektiven Zerstörung von Tumorzellen, während umgebendes Hirngewebe unverletzt blieb. ONYX-015 ist ein weiteres onkolytisches Virus, welches für die Glioblastomtherapie entwickelt wurde. Durch eine Deletion im E1B-Gen soll dieses Adenovirus Zellen mit defektem p53 selektiv lysieren.

Sowohl die onkolytischen HSV als auch ONYX-015 wurden bereits klinisch für die Behandlung von Gliomen getestet. Beide attenuierten onkolytischen Viren wiesen in klinischen Phase I/II Studien eine ausreichende Sicherheit auf. Unabhängig davon, ob man die Viren intratumoral oder in die Resektionshöhle injizierte, wurde die Behandlung gut vertragen und es konnten keine schwerwiegenden Nebenwirkungen beobachtet werden. Allerdings waren die cytolytischen Effekte nur von transienter Natur, stets gefolgt von einem Rezidiv (Cutter et al., 2006). Da in der Regel die Proliferationsrate von Gliomen die Amplifikationsrate und somit die Ausbreitungswelle der Viren übersteigt, ist die Vernichtung von Gliomen allein durch Onkolyse fraglich. Vielmehr ist für eine effiziente Behandlung die Kombination mehrerer Wirkprinzipien erforderlich. Durch Verwendung von Suizid- oder immunmodulatorischen Genen in OV konnte in präklinischen Untersuchungen eine synergistische Wirkung demonstriert werden (Tyminski et al., 2005; Fukuhara et al., 2005).

Neben den oben erwähnten DNA Viren, sind onkolytische RNA Viren ebenfalls in der Entwicklung. VSV ist ein umhülltes Negativ-Strang-Virus dessen Wirtsspektrum Nager und Nutztiere umfasst. Infektionen von Menschen sind selten und verlaufen meist asymptomatisch. Aufgrund der sehr geringen Seroprävalenz in der Bevölkerung ist eine Beeinträchtigung der Therapieeffizienz durch VSV-neutralisierende Antikörper nicht zu erwarten. Die Infektion und die zytoplasmatische Replikation von VSV finden unabhängig vom Zellzyklus statt, so dass teilungsaktive und ruhende Zellen gleichermaßen infiziert werden. Die effiziente und präferentielle Lyse von neoplastischen Zellen durch VSV hängt mit dem meist defekten Interferonsignalweg und der damit einhergehenden präferentiellen viralen Replikation in diesen Zellen zusammen (Wollmann et al., 2007). Auch konnte gezeigt werden, dass unabhängig von der zellulären Immunantwort, Tumorzellen mit Defekten in den Genen Myc, Ras oder p53 ebenfalls die Vermehrung von VSV unterstützen (Barber, 2004). Die Tumorspezifität von VSV konnte in den letzten Jahren durch die Herstellung rekombinanter Viren weiter optimiert werden. Das Hauptaugenmerk liegt dabei bei Varianten mit Mutation im M Protein (ΔM51). Diese Variante ist nicht in der Lage, die Interferonantwort in gesunden Zellen zu unterbinden, wodurch eine Replikation des Virus in solchen Zellen gehemmt wird. In Tumorzellen, mit defekter IFN-Antwort, kann das Virus replizieren und somit selektiv onkolytisch aktiv sein. Selbst nach systemischer Applikation zeigte VSV^{ΔM51} eine sichere und effiziente Onkolyse von humanen Gliomen im Mausmodell (Lun et al., 2006).

Die Applikation von viralen Vektoren direkt in das Gehirn erfordert eine hohe Selektivität für Tumorzellen und ist nur in relativ kleinen Volumina möglich. Ein effizienter Gentransfer kann daher nur mit sehr hoch konzentrierten Vektorpräparationen (>10⁸/ml), die einen ausgeprägten Tropismus für Gliomzellen ausweisen, erreicht werden. Bei gammaretro- und lentiviralen Vektoren können durch den Einbau eines nicht retroviralen Hüllproteins Vektortropismus und Vektorstabilität beeinflusst werden. Häufig wird das retrovirale Hüllprotein gegen das stabilere G-Protein von VSV ausgetauscht. Ein Problem dieser sogenannten Pseudotypvektoren ist, dass VSV-G zelltoxisch ist, und zwar sowohl in den Produzentenzellen als auch für das umliegende, gesunde Gewebe, was einem breiten Einsatz solcher VSV-G-Pseudotypen in der Klinik bisher im Wege stand.

Die Erfinder haben bereits einen retroviralen Vektortyp entwickelt, der einen effizienten Gentransfer in gliale Zellen des ZNS erlaubt. In diesem neuen Vektortyp dient das Glykoprotein GP des Lymphozytären Choriomeningitisvirus (LCMV) als virales Hüllprotein (Miletic et al., 1999; Beyer et al., 2002; EP 1 006 196). In vergleichenden *in vitro* und *in vivo* Tropismusstudien konnte gezeigt werden, dass LCMV-GP Pseudotypen präferentiell Gliomzellen transduzieren (Miletic et al., 2004; Miletic et al., 2007). Im Gegensatz dazu, wurden durch VSV-G-Pseudotypen insbesondere Neuronen transduziert, während der Gentransfer in Gliomzellen weniger effizient war als mit LCMV-GP-Pseudotypen. Auch einzelne infiltrierende Tumorzellen konnten durch LCMV-GP-Pseudotypen effizient transduziert werden. Im Rattengliom Modell konnten 90% der Ratten durch die intratumorale Injektion LCMV-GP pseudotypisierter Lentivektoren geheilt werden (Miletic et al., 2007b). Die verwendeten Vektoren kodierten für die Thymidinkinase (TK) des Herpes Simplex Virus, wodurch in den transduzierten, aber auch umliegenden Zellen, Ganciclovir in eine zelltoxische Triphosphatverbindung umgewandelt wird.

Ausschlaggebend für den Therapieerfolg ist nicht nur der selektive Gentransfer in Gliome, sondern auch eine effiziente Vektorverteilung im gesamten Tumor. Die Erfinder haben daher tumorinfiltrierender Verpackungszellen, die im gesamten Tumor pseudotypisierte Vektoren freisetzen sollen, entwickelt (WO 2006/008074). Ein vielversprechender Zelltyp mit migratorischen Fähigkeiten ist die multipotente adulte Vorläuferzelle, die aus dem Knochenmark isoliert werden kann (Jiang et al., 2002). In Transplantationsexperimenten wurde mit Hilfe des Rattengliommodells das Migrationsverhalten dieser Zellen untersucht. Dabei konnte gezeigt werden, dass die Vorläuferzellen effizient die Tumormasse durchdrangen, aber nicht umliegendes gesundes Gehirngewebe infiltrierten (Fischer et al., 2007). Gängige Zelllinien wie 3T3 Mausfibroblasten, Rat-1 Rattenfibroblasten oder humane 293T zeigten keine Tumorinfiltration. Vielmehr waren diese Zellen lokal auf die Injektionsstelle, oder auf die nähere Umgebung beschränkt und zeigten keine gliomspezifische Migration.

In einer weiteren Studie untersuchten die Erfinder die therapeutische Wirksamkeit von TK-exprimierenden Vorläuferzellen im Rattengliommodell. Das Ergebnis dieser Studie war, dass es alleine aufgrund des "bystander effects" zwischen Vorläufer- und Tumorzelle zur Zerstörung des Tumors in 70% der Ratten kam (Miletic et al., 2007). Zudem konnte die intratumorale Lokalisation der Vorläuferzellen mittels bildgebender Verfahren bestätigt werden. Histologische Gehirnschnitte von behandelten, symptomfreien Ratten wiesen an der Stelle des Tumors eine Aushöhlung mit ausgeprägtem Narbengewebe auf, ein Hinweis darauf, dass durch die Gentherapie der Tumor in den Tieren erfolgreich eliminiert wurde. Dank des starken Expansionspotentials dieser Vorläuferzellen ist eine genetische Modifikation mit anschließender Selektion einzelner Verpackungszellklone möglich. Es wurde eine Vorläuferzell-basierte Verpackungszelle für gammaretrovirale LCMV-GP Pseudotypen entwickelt. Diese Verpackungszellen produzierten konstant retrovirale Vektoren mit einem Titer von 1-7x10E3 TU/ml. Die Titer blieben stabil über mehrere Wochen und nach mehrmaligem Einfrieren und Auftauen der Zellen (Fischer et al., 2007).

Der ineffiziente Gentransfer *in vivo* und nicht der Mangel an therapeutisch effektiven Genen steht derzeit einer erfolgreichen Gentherapie des Glioblastoms entgegen. Die bisher bekannten Vektoren zur Gentherapie und der onkolytischen Virotherapie von Gliomen sind aus verschiedenen Gründen nicht optimal. Die Effizienz, Spezifität und Sicherheit bisheriger Gentransferverfahren soll soweit gesteigert werden, dass ein therapeutisch effektiver Gentransfer im Patienten möglich wird.

Die Aufgabe der vorliegenden Erfindung ist daher ein hochpotentes onkolytisches virales Gentransfersystem für therapeutische Gene zur Therapie hochmaligner Hirntumore wie Gliome und anderer solider Tumoren zu entwickeln.

### ZUSAMMENFASSUNG DER ERFINDUNG

Gegenstand der Erfindung ist daher ein Vesikular Stomatitis Virus (VSV)-Pseudotypvektor, welcher ein für ein Glykoprotein GP des Lymphozytären Choriomeningitisvirus (LCMV) kodierendes Gen und kein funktionsfähiges Gen für Hüllprotein G des VSV enthält. Im Folgenden wird der erfindungsgemäße Vektor "VSV-LCMV-GP-Pseudotypvektor" genannt. GP des LCMV (LCMV-GP) kann das Glycoprotein GP-1 oder GP-2 des LCMV sein.

Nach einem weiteren Merkmal ist im VSV-LCMV-GP-Pseudotypvektor das Hüllprotein G des VSV durch LCMV-GP ersetzt.

Nach einem weiteren Merkmal fehlt auf dem Vektor mindestens ein aus der Gruppe von für N-, L- P- und M-Protein des VSV kodierenden Gene *n, l, p* und *m* ausgewähltes Gen.

Nach einem weiteren Merkmal enthält das M-Protein des VSV Mutationen, die die Zytopathogenität des VSV reduzieren. Beispiele für die Zytopathogenität des VSV reduzierende Mutationen sind Aminosäureaustausche in der ³⁷PSAP⁴⁰ Region des M-Proteins sowie Mutationen M33A, M51A, V221F, S226R oder deren Kombination.

Nach einem weiteren Merkmal enthält der VSV-LCMV-GP-Pseudotypvektor mindestens ein therapeutisch anwendbares Transgen. Das Transgen kann ein Suizidgen oder ein immunstimulatorisches Gen sein. Beispiele für Suizidgene sind Gene, die für Thymidinkinase des Herpes-Simplex-Virus (HSV-TK), Cytosin-Deaminase, FKBP-FAS oder FKBP-caspase9 kodieren. Beispiele für immunstimulatorische Gene sind Gene, die für Cytokine IL-2, IL-4, IL-12, neutralisierendes anti-TGFbeta oder Flt3L kodieren.

Nach einem weiteren Merkmal enthält der VSV-LCMV-GP-Pseudotypvektor ein Markergen. Das Markergen kann LacZ, ein Antibiotikaresistenzgen oder ein für Fluoreszenzprotein (GFP, RFP, GGP usw.) kodierendes Gen sein.

Gegenstand der Erfindung ist ferner ein VSV-LCMV-GP-Pseudotypvektorsystem, welches mindestens zwei komplementierend replizierende (kr) VSV-Vektoren umfasst, wobei ein Vektor des Vektorsystems ein für LCMV-GP kodierendes Gen *gp* enthält, wobei das Vektorsystem ferner für N-, L- P- und M-Protein des VSV kodierende Gene *n*, *l, p* und *m* und kein funktionsfähiges Gen für Hüllprotein G des VSV enthält, wobei jedem Vektor des Vektorsystems eins der *gp*, *n, l, p* und *m* Gene ("komplementierendes Gen") fehlt, und wobei das fehlende Gen auf einem anderen Vektor des Vektorsystems vorhanden ist. Bevorzugt als komplementierende Gene sind *m* und *gp*.

Nach einem weiteren Merkmal enthält das M-Protein des VSV-LCMV-GP-Pseudotypvektorsystems die Zytopathogenität des VSV reduzierende Mutationen, wie oben dargestellt.

Nach einem weiteren Merkmal enthält das VSV-LCMV-GP-Pseudotypvektorsystem mindestens ein therapeutisch anwendbares Transgen und/oder ein Markergen, wie oben dargestellt. Das Transgen/Markergen kann sich auf einem beliebigen Vektor des Vektorsystems befinden.

Ein weiterer Gegenstand der Erfindung ist ein mit LCMV-GP pseudotypisiertes VSV-Virion, welches ein Glykoprotein GP des LCMV als Hüllprotein enthält.

Ein weiterer Gegenstand der Erfindung ist eine Virusproduzentenzelle, welche ein mit LCMV-GP pseudotypisiertes VSV-Virion produziert.

Nach einem weiteren Merkmal ist Virusproduzentenzelle eine adulte Stammzelle. Die adulte Stammzelle kann eine multipotente adulte Progenitorzelle (MAPC), eine neuronale Stammzelle (NSC), eine mesenchymale Stammzelle (MSC) oder eine von MSC abgeleitete BM-TIC Zelle (*b*one *m*arrow *d*erived *t*umor *i*nfiltrating *c*ell) sein.

Nach einem weiteren Merkmal enthält die Virusproduzentenzelle eine oder mehrere Expressionskassetten für die Expression von Genen ausgewählt aus der Gruppe bestehend aus für N-, L-, P- und M-Proteine des VSV kodierenden Gene *n, l, p* und *m* sowie einem für LCMV-GP-Glykoprotein kodierenden Gen *gp*.

Nach einem weiteren Merkmal enthält die Virusproduzentenzelle ferner einen Gentransfervektor zur Verpackung in ein mit LCMV-GP pseudotypisiertes VSV-Virion.

Nach einem weiteren Merkmal enthält der Gentransfervektor ein therapeutisch anwendbares Transgen und/oder ein Markergen, wie oben dargestellt.

Ein weiterer Gegenstand der Erfindung ist ein *in vitro* Verfahren zum Transfer eines Transgens in eine Zelle, bei dem man die Zelle mit einem mit LCMV-GP pseudotypisierten VSV-Virion transduziert, wobei das Virion ein Transgen enthält.

Ein weiterer Gegenstand der Erfindung ist ein *in vitro* Verfahren zum Transfer eines Transgens in eine Zelle, bei dem man die Zelle mit einer Virusproduzentenzelle kontaktiert, welche ein mit LCMV-GP pseudotypisiertes VSV-Virion produziert, wobei das Virion ein Transgen enthält.

Es ist bevorzugt, dass die Zelle eine Tumorzelle, zum Beispiel eine Gliomzelle, ist.

Ein weiterer Gegenstand der Erfindung ist die Verwendung eines VSV-LCMV-GP-Pseudotypvektors oder eines VSV-LCMV-GP-Pseudotypvektorsystems der Erfindung zur Herstellung einer pharmazeutischen Zusammensetzung zur Therapie eines soliden Tumors.

Ein weiterer Gegenstand der Erfindung ist die Verwendung eines mit LCMV-GP pseudotypisierten VSV-Virions der Erfindung zur Herstellung einer pharmazeutischen Zusammensetzung zur Therapie eines soliden Tumors.

Ein weiterer Gegenstand der Erfindung ist die Verwendung einer Virusproduzentenzelle der Erfindung zur Herstellung einer pharmazeutischen Zusammensetzung zur Therapie eines soliden Tumors. Bevorzugt ist ein Hirntumor, insbesondere ein Gliom.

Nach einem weiteren Merkmal werden mindestens zwei Virusproduzentenzellen zur Herstellung einer pharmazeutischen Zusammensetzung zur Therapie eines soliden Tumors verwendet, wobei eine erste Virusproduzentenzelle einen ersten Vektor des VSV-LCMV-GP-Pseudotypvektorsystems und eine zweite Virusproduzentenzelle einen zweiten Vektor des VSV-LCMV-GP-Pseudotypvektorsystems enthält.

Gegenstand der Erfindung ist ferner eine pharmazeutische Zusammensetzung, welche einen VSV-LCMV-GP-Pseudotypvektor, ein VSV-LCMV-GP-Pseudotypvektorsystem, ein mit LCMV-GP pseudotypisiertes VSV-Virion oder eine Virusproduzentenzelle, welche ein mit LCMV-GP pseudotypisiertes VSV-Virion produziert, enthält. Die Zusammensetzung kann auch geeignete Hilfsstoffe und/oder Träger enthalten.

### BESCHREIBUNG DER FIGUREN

- Figur 1:: Pseudotypisierung von VSVgfp ncp-ΔG-Vektoren durch LCMV-GP
- Figur 2:: Transduktion von Gliomzelllinien mit VSV-LCMV-GP-Vektoren
- Figur 3:: Pseudotypisierung von VSVgfp ncp-ΔG-Vektoren durch LCMV GP in BM-TIC
- Figur 4:: Schematische Darstellung des kr VSV-LCMV-GP-Vektorsystems. a) Einer der Vektoren enthält rfp anstelle des Phosphoproteins P und LCMV-GP anstelle des VSV-G. b) Der komplementierende Vektor kodiert für das in a) fehlende P-Protein, enthält aber anstelle eines viralen Glykoproteins beispielsweise die Thymidinkinase (TK) des Herpes Simplex Virus als therapeutisches Gen. Beide Vektoren enthalten ein modifiziertes M-Gen (Mncp).
- Figur 5:: Schematische Darstellung der viralen Vektoren. In den optimierten Vektor soll anstelle des P-Proteins das therapeutische Gen kloniert werden. Neben den immunmodulatorischen Genen (IL-12, Flt3L) enthält der zweite Vektor zusätzlich die Thymidinkinase (TK) von HSV.

### BESCHREIBUNG DER ERFINDUNG

Die Erfindung bezieht sich auf rekombinante Vesikular Stomatitis Viren (VSV) und VSV-Vektoren. Das VSV-Genom enthält fünf für die Vermehrung des Virus essentielle Gene 1, m, n, p und g, die für die Proteine L, M, N, P und G kodieren. N ist ein Nukleoprotein, das die VSV genomische RNA verpackt; nur als RNA-Protein-Komplex kann das VSV-Genom repliziert werden. L und P bilden zusammen einen Polymerasekomplex, der das VSV-Genom repliziert und die VSV mRNA transkribiert. M ist ein Matrixprotein, das im Viruspartikel eine Art Kit zwischen Lipidhülle und Nukleokapsid bildet und für die Partikelsprossung an der Zellmembran wichtig ist. G ist das Hüllprotein, das in virale Hülle eingebaut wird und für Infektiosität des Virus essentiell ist.

Gegenstand der Erfindung ist ein VSV-LCMV-GP-Pseudotypvektor und ein mit LCMV-GP pseudotypisiertes VSV-Virion. Der Vektor der Erfindung enthält ein für das GP-Protein des LCMV kodierendes Gen *gp* anstatt des für das G-Protein des VSV kodierenden Gens *g*. Dementsprechend enthält das Virus/Virion der Erfindung ein LCMV-GP-Protein als Hüllprotein. Das LCMV-GP-Protein kann GP1 oder GP2 sein. Die Erfindung schließt GP-Proteine aus verschiedenen LCMV-Stämmen ein. Insbesondere können sich LCMV-GP-Varianten von LCMV-Wildtyp oder LCMV-Stämmen LCMV-WE, LCMV-WE-HPI, LCMV-WE-HPIopt ableiten (WO 2006/008074).

Vektoren, die auf dem Virus der vesikulären Stomatitis beruhen, haben mehrere Vorzüge gegenüber retroviralen Vektoren:
(i) VSV-Vektoren sind onkolytisch und haben im Vergleich zu anderen onkolytischen, viralen Vektoren eine besonders hohe onkolytische Aktivität.
(ii) VSV-Vektoren replizieren präferentiell in Tumorzellen und haben im Vergleich zu anderen onkolytischen, viralen Vektoren eine besonders hohe Replikationsfähigkeit.
(iii) VSV-Vektoren infizieren teilungsaktive als auch ruhende Tumorzellen.
(iv) VSV-Vektoren induzieren eine starke angeborene (innate), humorale und zelluläre Immunantwort.
(v) VSV-Vektoren replizieren rein zytoplasmatisch, können also als RNA-Viren weder ins Wirtszellgenom integrieren noch zu replikationskompetenten Viren rekombinieren.
(vi) VSV-Vektoren sind einfach zu verpacken.
(vii) Das VSV-Glykoprotein ist gegen ein fremdes Hüllprotein austauschbar. Beispiele für Glykoproteine, die bisher in die VSV-Hülle eingebaut wurden, sind: HIVgp160 (Owens and Rose 1993), HCVE1/E2 (Tani et al., 2007), SARS S (Ge et al., 2006) und Lassa GP (Garbutt et al., 2004).

Zusammengenommen haben die VSV-Vektoren ein besonders großes therapeutisches Potential.

Ein weiterer Vorteil des erfindungsgemäßen VSV-LCMV-GP-Pseudotypvektors liegt in einer erheblich reduzierten Toxizität der mit LCMV-GP pseudotypisierten VSV-Viren gegenüber gesunden Hirnzellen, d.h. Neuronen. Die Neurotoxizität des VSV ist auf das G-Protein des VSV zurückzuführen (Shinosaki et al., 2005). Eigentlich infizieren VSV-G Pseudotypen bevorzugt normale Neuronen. Da für alle Anwendungen von onkolytischem VSV der Neurotropismus dosislimitierend ist, ist die Verwendung des erfindungsgemäßen Vektors bei allen Tumoren von großem Vorteil.

Außerdem hat der erfindungsgemäße VSV-LCMV-GP-Pseudotypvektor eine erhöhte Spezifität für Hirntumorzellen, die sich vom gliomspezifischen Tropismus des verwendeten LCMV-GP-Glykoproteins und von der selektiven Transkription/Replikation von VSV in Tumorzellen ableitet. Im Gegensatz zu VSV-G weist das LCMV-GP Hüllprotein einen besonderen Tropismus für Glioblastomzellen auf. Allerdings kann der erfindungsgemäße Vektor auch bei anderen Tumoren außerhalb des ZNS erfolgreich angewendet werden, da auch dort die Neurotoxizität von VSV, insbesondere bei systemischer Applikation, dosislimitierend ist.

Mit LCMV-GP pseudotypisierte Vektoren haben drei entscheidende Eigenschaften, die nicht pseudotypisierte Vektoren nicht haben, nämlich:
(i) Das LCMV-GP ist nicht zelltoxisch.
(ii) Die LCMV-GP Pseudotypvektoren lassen sich ohne Verlust der Infektiosität durch Ultrazentrifugation konzentrieren.
(iii) Die LCMV-GP Pseudotypvektoren zeigen einen bevorzugten Tropismus für gliale Zellen, während Neuronen ineffizient infiziert werden.

Die Erfindung schließt sowohl replikationsdefiziente als auch replikationskompetente Viren ein. Die letzteren weisen einen zusätzlichen Vorteil auf, da durch die Verwendung vermehrungsfähiger Viren eine hohe Transduktionsrate erreicht werden kann. Insofern sind replikationsfähige onkolytische virale Vektoren effizienter als replikationsinkompetente Vektoren.

Zu Erhöhung der Sicherheit bei der Verwendung replikationsfähiger Viren in therapeutischen Einsetzen wird ein Vektorsystem zur Verfügung gestellt, welches gewährleistet, dass Replikation, Onkolyse und die Produktion der VSV-Viren nur in Zellen stattfindet, die durch mindestens zwei replikationsdefiziente, aber sich ergänzende Vektoren infiziert wurden.

Gegenstand der Erfindung ist somit ein VSV-LCMV-GP-Pseudotypvektorsystem, das mindestens zwei sich komplementierende VSV-Vektoren enthält. Solche komplementierend replizierende (kr) VSV-Vektoren können sich bedingt innerhalb des Tumorgewebes ausbreiten, was die Effizienz des Gentransfers und der Onkolyse steigert. Somit ermöglicht das erfindungsgemäße Vektorsystem die Herstellung onkolytischer VSV-LCMV-GP-Pseudotypvektoren mit begrenzter Vermehrungsfähigkeit zur Genübertragung in Gliomen und anderen soliden Tumoren.

Das Prinzip des erfindungsgemäßen Vektorsystems besteht darin, dass auf jedem Vektor des Systems eins der essentiellen Gene *m, n, l* und *p* des VSV oder *gp* des LCMV fehlt, welches aber auf einem anderen Vektor des Systems vorhanden ist. Das für LCMV-GP kodierende Gen *gp* sowie eventuelle zusätzliche Gene wie Therapie- und/oder Markergene können auf jedem beliebigen Vektor des Systems vorliegen.

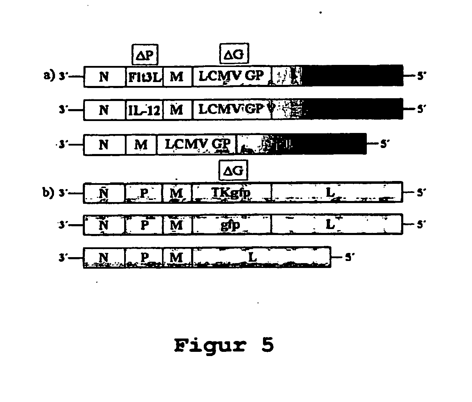

Es sind verschiedene Variante des erfindungsgemäßen Vektorsystem möglich. Zum Beispiel kann das Vektorsystem aus zwei Vektoren bestehen, wie in Figur 5 dargestellt ist. Ein erster Vektor enthält GP des LCMV anstelle von G des VSV und eine Deletion des für P-Protein kodierenden Gens *p*. Ein zweiter Vektor enthält ebenfalls kein VSV-G, exprimiert aber das P-Protein des VSV. Jeder Vektor exprimiert Nukleoprotein (N) und Polymerase (L) des VSV sowie eine weniger zytopathogene Variante des M-Proteins (M_{ncp}). Der erste Vektor trägt außerdem das Markergen *rfp,* während der zweite Vektor das Suizidgen *HSV-TK* und das Markergen *gfp.* In den in Figur 6 dargestellten Varianten können beide Vektoren ein therapeutisches Gen enthalten, wobei der erste Vektor z.B. *Flt3L* oder *IL-12* und der zweite Vektor z.B. *HSV-TK* enthält.

Zur Therapie des Glioblastoms werden bislang verschiedene Konzepte verfolgt: *(i)* die Übertragung von Suizidgenen, *(ii)* die Aufhebung der glioblastombedingten Immunsuppression anhand immunstimulatorischer Gene und mittels zytokinbasierender Immuntherapie, *(iii)* die Übertragung von Faktoren, die der tumorinduzierten Angiogenese entgegenwirken, *(iv)* die Anwendung von Zellzyklusmodulatoren und *(v)* die Induktion von Apoptose. Für erfindungsgemäße Zwecke kommen vor allem die drei erstgenannten Ansätze *(i-iii)* in Betracht.

Die erfindungsgemäßen VSV-LCMV-GP-Pseudotypvektoren und darauf basierenden Vektorsysteme können daher zusätzlich zu ihren inhärenten Onkolyse-Eigenschaften durch das Einbringen von Suizidgenen oder/und immunstimulatorischen Genen weiter verbessert werden. Beispiele für Suizidproteine sind Thymidinkinase des Herpex Simplex Virus (HSV-TK), cytosine deaminase, FKBP-FAS, FKBP-caspase9. Beispiele für immunstimulatorische Proteine sind Cytokine wie IL-2, IL-4, IL-12 sowie Flt3L, neutralizing anti-TGFbeta. Insertionen von bis zu 4,5 kb großen Genen werden vom VSV-Genom toleriert, sodass es sogar möglich wäre, zwei oder mehr therapeutische Gene (z.B. HSV-TK + Zytokin) auf einem VSV-Genom zu vereinen. Außerdem wirken die Genprodukte auch auf nicht infizierte Tumorzellen mittels eines sogenannten Bystander-Effekts.

Injizierte, virale Vektoren dringen von der Einstichstelle nur wenige Millimeter in das Tumorgewebe ein. Zur Optimierung der Vektordistribution und zur gezielten Zerstörung des Tumors werden tumorinfiltrierende Virusproduzentenzellen verwendet, die spezifisch im Tumor migrieren und so die Viren an von der Injektionsstelle entfernter Stelle freisetzen.

Virusproduzentenzellen im Sinne der Erfindung schließen sowohl klassische Verpackungszellen zur Produktion von Virionen aus replikationsunfähigen Vektoren als auch Produzentenzellen zur Produktion von Virionen aus vermehrungsfähigen Vektoren ein. Verpackungszellen enthalten üblicherweise eine oder mehreren Plasmiden zur Expression von essentiellen Genen, welche auf dem jeweiligen zu verpackenden Vektor fehlen und/oder zur Produktion von Virionen notwendig sind.

In bisherigen Studien wurden zwar Verpackungszellen zur Übertragung viraler Vektoren verwendet, es handelte sich hierbei jedoch hauptsächlich um Fibroblasten, die im Tumor nicht migrieren (Short et al., 1990, Culver et al., 1992). Adulte Stammzellen, insbesondere neuronale (NSC) und mesenchymale Stammzellen (MSC), hingegen haben ein hohes migratorisches Potential. Sie bleiben auf das Tumorgewebe beschränkt, wodurch ein sehr effizienter aber auch spezifischer Gentransfer in das Tumorgewebe erreicht wird. Allerdings haben diese Stammzellen die begrenzte Passagierbarkeit *in vitro.*

Eine Subpopulation adulter mesenchymaler Stammzellen, sogenannte BM-TIC (*b*one *m*arrow *d*erived *t*umor *i*nfiltrating *c*ells), infiltrieren nach Injektion in experimentell induzierte Gliome den gesamten Tumor und verfolgen darüber hinaus einzelne, von der Tumormasse abgelegene Tumorzellen (Miletic et al., 2007). BM-TIC werden aus adultem Knochenmark isoliert, haben ein hohes Expansionspotential und können als migrierende Produzentenzellen für MLV (Fischer et al., 2007) und VSV-Vektoren verwendet werden.

Gegenstand der Erfindung sind daher Virusproduzentenzellen, die onkolytische VSV-LCMV-GP-Pseudotypvektoren produzieren. Insbesondere sind es tumorinfiltrierende Produzentenzellen, welche während ihrer Migration im Tumor die genannten Vektoren freisetzen. Bevorzugt sind adulte Stammzellen, insbesondere neuronale (NSC) und mesenchymale Stammzellen (MSC). Besonders bevorzugt sind von MSC abgeleitete BM-TIC Zellen.

Ein Hindernis für die Herstellung einer rhabdoviralen Virusproduzentenzelle stellt die Zytopathogenität der Proteine M und G dar. Da in erfindungsgemäßen VSV-LCMV-GP-Pseudotypvektoren das G Protein des VSV durch das gliomspezifische und nicht zytotoxische Glykoprotein des LCMV ersetzt wird, stellt allein das M-Protein ein Problem dar. Zur Reduzierung der Toxizität des M-Proteins in der VSV-Produzentenzelllinie kann eine nicht zytopathogene Variante des M Proteins verwendet werden.

Die erfindungsgemäßen Virusproduzentenzellen und dementsprechend auch die von diesen produzierten VSV-LCMV-GP-Pseudotypvektoren können dementsprechend ein für mutiertes M-Protein kodierenden Gen enthalten. Diese Vektorvariante ist selektiv onkolytisch für Tumorzellen, während sie für gesunde Zellen nicht toxisch ist. Bevorzugt sind M-Varianten mit Aminosäurenaustausch in der ³⁷PSAP⁴⁰ Region des M-Proteins oder mit einfacher (M51R) oder mehrfachen (V221F und S226R; M33A und M51A) Mutationen außerhalb der PSAP Region des M-Proteins. Besonderes bevorzugt ist ein M-Protein mit Mutationen M33A, M51R, V221F und S226R enthält. Um eine effiziente Virusproduktion in Verpackungszellen zu gewährleisten, kann die M-Variante mit einem viralen Interferonantagonisten stabil transfiziert werden.

Außerdem ist Gegenstand der Erfindung ein *in vitro* Verfahren zum Gentransfer, wobei ein VSV-LCMV-GP-Pseudotypvektor oder VSV-LCMV-GP-Pseudotypvektorsystem, der/das ein Transgen enthält, in eine Zelle direkt oder mit Hilfe erfindungsgemäßer Virusproduzentenzellen (Verpackungszellen) eingeführt wird. Im Fall der Verwendung eines komplementierend replizierenden (kr) Vektorsystems mit mindestens zwei Vektoren werden mindestens zwei Verpackungszellen eingesetzt, die jeweils einen der (replikationsunfähigen) kr Vektoren produzieren. Nur Zellen, die mit allen Vektoren des kr Vektorsystems infiziert sind und dementsprechend alle essentiellen viralen Gene enthalten, findet die Produktion von VSV-Viren statt.

Die Erfindung bezieht sich außerdem auf die Verwendung von erfindungsgemäßen Vektoren und Virusproduzentenzellen als Medikamente in therapeutischen Verfahren. Insbesondere werden die erfindungsgemäßen Vektoren und Virusproduzentenzellen für die Therapie solider Tumoren eingesetzt. Der therapeutische Effekt wird sowohl durch die onkolytischen Eigenschaften der rekombinanten Vektoren und Viren als auch durch den Einsatz therapeutischer Gene bewirkt.

Solide Tumoren können Hirntumor, Lebertumor, insbesondere hepatozelluläres Karzinom, Lungentumor, insbesondere Bronchialkarzinom, und Darmtumor, insbesondere Kolonkarzinom, sein. Bevorzugte Tumore sind Hirntumore, zum Beispiel ein Gliom, insbesondere Ependymom, Oligodendrogliom, Oligoastrozytom, Astrozytom, Glioblastom, oder ein Medulloblastom.

Gegenstand der Erfindung ist weiter eine pharmazeutische Zusammensetzung, die den Vektor/das Virion/die Virusproduzentenzelle der Erfindung und eventuell Zusätze wie Träger- und Hilfsstoffe enthält.

Um die virale Onkolyse und die Übertragungseffizienz der therapeutischen Gene zu erhöhen, werden tumorinfiltrierende Virusproduzentenzellen, die kontinuierlich Vektoren freisetzen, zur direkten Implantation in die Tumoren formuliert.

Die Erfindung wird anhand folgender Beispiele erläutert.

### BEISPIELE

### Beispiel 1: Transduktion von Gliomazellen durch VSV-LCMV-GP-Pseudotypvektoren

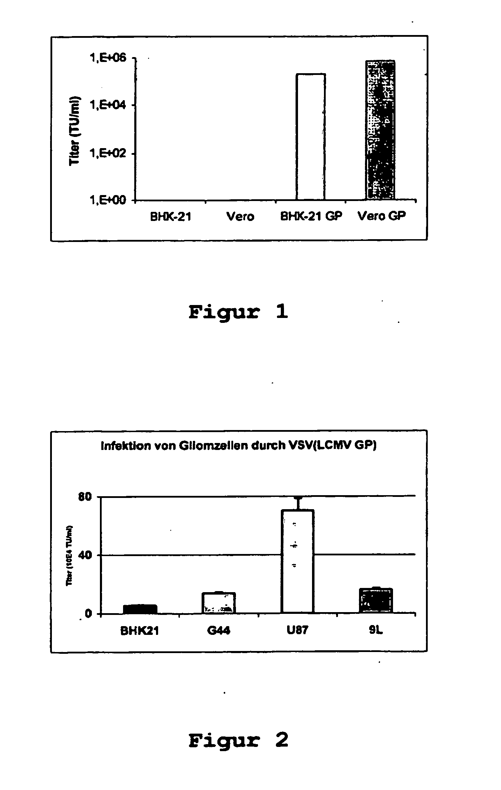

Mit defekten VSV-Vektoren, die GFP aber kein virales Hüllprotein kodieren (VSVgfp-ncp-ΔG), wurden BHK-21 und Vero-Zellen transduziert, die wiederum stabil das GP von LCMV exprimieren. 1x105 Zellen (BHK-21, -GP, Vero, -GP) wurden pro 24well Vertiefung ausgesät und vier Stunden später mit einer MOI=5 mit VSVgfp-ncp-ΔG-Vektoren transduziert. 24 Stunden später wurde der Kulturüberstand gesammelt und der Titer mittels FACS Analyse der GFP-Expression in BHK21 bestimmt. Figur 1 zeigt, dass die transduzierten Zellen LCMV-GP pseudotypisierte VSV-Vektoren produzierten, wobei je nach verwendetem Zelltyp die Pseudotyptiter zwischen 2-7 x10E5 TU/ml variierten.

Da die Pseudotypen für den Gentransfer in Gliome genutzt werden sollen, wurde die Transduktionseffizienz der Vektoren bei verschiedenen Gliomzelllinien untersucht. Zwei humane Gliomzelllinien (U87, G44) und eine Rattengliomzelllinie (9L) wurden getestet. Als Kontrolle dienten erneut BHK-21 Zellen. 1x105 Zellen pro 24well Vertiefung wurden ausgesät und vier Stunden später mit einer MOI=0,3 (Titration auf BHK21) mit VSV-LCMV-GP-Pseudotypvektoren transduziert. 24 Stunden später wurde der Prozentsatz GFP-exprimierender Zellen durch FACS Analyse bestimmt und der Titer daraus errechnet. Figur 2 zeigt, dass die Gliomzelllinien effizient durch LCMV-GP-pseudotypisierte VSV-Vektoren transduziert wurden.

### Beispiel 2: Verpackung VSV-LCMV-GP-Pseudotypvektoren in Multipotenten Vorläuferzellen

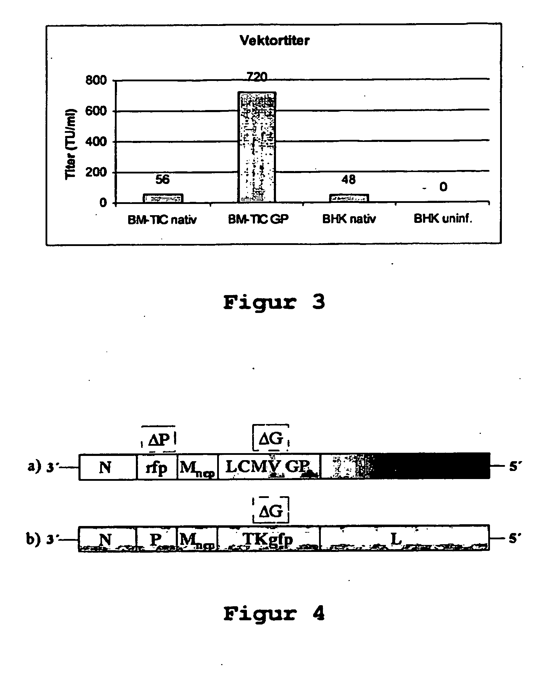

Die Erfinder haben bereits gezeigt, dass Stammzellen, die nach dem Protokoll von C. Verfaillie isoliert wurden (Jiang et al., 2002), tumorinfiltrierende Eigenschaften besitzen. Da nur ihr Migrations- aber nicht ihr Differenzierungspotential untersucht wurde, wurden diese Zellen BM-TIC (bone *m*arrow derived tumor infiltrating cells) genannt. Zur Testung der Verpackungsfähigkeit von BM-TIC für VSV-Vektoren wurden LCMV-GP exprimierende BM-TIC mit dem defekten VSV-GFP-Vektor transduziert. 1x105 Zellen (BM-TIC, -GP, BHK-21) wurden pro 24well Vertiefung ausgesät und vier Stunden später mit einer MOI=5 mit VSVgfp-ncp-ΔG-Vektoren transduziert. 24 Stunden später wurde der Kulturüberstand gesammelt und auf BHK-21 Zellen titriert. Figur 3 zeigt, dass VSV-LCMV-GP-Pseudotypen auch in BM-TIC herstellbar sind. Allerdings sind die Titer geringer als beispielsweise mit BHK-21 GP Zellen (siehe Figur 1).

### Beispiel 3: Entwicklung von komplementierend replizierenden (kr) VSV-LCMV-GP-Pseudotypvektoren

Eine hohe Transduktionsrate lässt sich mit vermehrungsfähigen Viren erreichen. Um die Gentransferrate in Gliome zu erhöhen, aber gleichzeitig ein hohes Maß an Sicherheit zu gewährleisten, wurde ein begrenzt replikationsfähiges VSV-Pseudotypvektorsystem etabliert. Für Retroviren (Trajcevski et al., 2005) und verschiedene Flaviviren (Riepl & Mandl, 2007) sind solche Replikationssysteme mit komplementär defekten Viren beschrieben worden. Das Prinzip solcher Systeme besteht darin, das virale Genom auf zwei inkomplette Replikons aufzuteilen. Infektiöse Viren werden nur gebildet, wenn ein und dieselbe Zelle mit beiden Vektoren infiziert ist und erst durch diese Koinfektion alle notwendigen Komponenten zum Verpacken des Virusgenoms enthält.

Abbildung 4 zeigt ein Beispiel der sich ergänzenden Vektoren des erfindungsgemäßen Vektorsystems. Für eine effiziente Replikation und Verpackung fehlt dem ersten Vektor das Phosphoprotein P. Um den viralen Titer dieser Vektoren einfacher bestimmen zu können, kodiert der Vektor anstelle von P das rotfluoreszierende Protein (rfp). Die fehlende P-Funktion wird bei Koinfektion der Zelle durch den' zweiten Vektor komplementiert. Dieser ist jedoch alleine nicht vermehrungsfähig, da ihm das essentielle Hüllprotein VSV-G fehlt. Mit Hilfe dieses Vektors werden therapeutische Gene appliziert.

Während allein die genomische RNA von Plusstrang-RNA-Viren infektiös ist, benötigen Negativstrang-RNA-Viren wie das VSV neben dem viralen RNA-Genom noch mindestens virales Nucleoprotein (N) und virale Polymerase (L), um aus klonierten viralen cDNAs ein infektiöses Virion zu generieren. Das System zur Herstellung rekombinanter VSV basiert auf der zytoplasmatischen Expression von viraler (+)RNA und den viralen Proteinen N, P und L mit Hilfe der T7-RNA-Polymerase, die konstitutiv in BSR T7/5 Zellen exprimiert wird. Um die gewünschten Vektoren herzustellen, werden genomische Konstrukte zusammen mit den Expressionsplasmiden für N, P und L und zusätzlich für VSV-G im Fall von Vektor b) (Figur 4) in die BSR T7/5 kotransfiziert. Durch Passagieren der so gewonnenen Viren auf Zellen, die die fehlende Komponente in trans exprimieren, kann man die Titer beachtlich erhöhen. Für den LCMV-GP defizienten Vektor wurde eine BHK-21-Zelllinie, die stabil GP exprimiert, etabliert. Für den P defizienten Vektor wurde eine BHK-21-Zelllinie, die das P-Protein exprimiert, etabliert.

### Herstellung und Charakterisierung von kr VSV-LCMV-GP-pseudotypisierten VSV-Vektoren

Nach Charakterisierung der einzelnen Vektoren hinsichtlich ihres Titers und ihrer Transduktionseffizienz wird die Ausbreitung der Vektoren *in vitro* untersucht. Dazu werden BHK-21-Zellen mit beiden Vektoren koinfiziert und die Replikation und somit die Entstehung neuer Vektoren durch serielle Passage des Kulturüberstandes auf nativen BHK-21 untersucht. Diese Experimente werden mit verschiedenen Glioblastomzelllinien (G44, G62, U87) wiederholt, die als Monolayer aber auch als Sphäroide kultiviert werden.

Tumorsphäroide sind dreidimensionale, organoide Zellverbände, die Beschaffenheit und Heterogenität von Tumoren besser widerspiegeln als Monolayerkulturen. Am Rande des Zellaggregates liegen die teilungsaktiven Tumorzellen im losen Verband, während tieferliegende Zellen sich nicht mehr teilen und es hier auch zur Ausbildung nekrotischer Areale kommt (Carlsson et al., 1984). Die Shpäroidexperimente geben über die intratumorale Transduktionseffizienz, die Ausbreitungkinetik und die onkolytische Wirkung des kr VSV-LCMV-GP-Pseudotypvektorsystems Aufschluss. Diese Spheroide ermöglichen somit eine initiale Optimierung des Systems, ohne Tierversuche durchführen zu müssen. Eine mögliche Toxizität der kr LCMV-GP-pseudotypisierten VSV-Vektoren, die therapeutische Wirksamkeit der Onkolyse und der Verbleib der Viren nach Tumorelimination werden im Tiermodell untersucht (siehe Beispiel 6).

Die virale RNA-Polymerase verfügt über keine Rekombinaseaktivität und es befinden sich auch keine zellulären Rekombinasen im Zytoplasma, so dass die Rekombination zwischen zwei Negativstrang-RNA-Viren nur durch einen Matrizenwechsel der RNA-Polymerase zustande kommen kann. Dies ist jedoch ein äußerst seltenes Ereignis (Finke & Conzelmann, 2005; Spann et al., 2003). Die Entstehung eines rekombinanten, replikationsfähigen VSV stellt ein Sicherheitsrisiko dar und wird überprüft. Dabei wird zu unterschiedlichen Zeitpunkten virale RNA aus dem Kulturüberstand koinfizierter Zellen isoliert und mit einer transgenspezifischen (z.B. gfp) Sonde im Northernblot untersucht. Sollten RNA Spezies mit "Überlänge" nachweisbar sein, werden die Viren aus dem Überstand plaquegereinigt und mittels Standardverfahren auf ihre Infektiosität untersucht und anschließend molekularbiologisch charakterisiert.

### Beispiel 4: Etablierung einer migrierenden Produzentenzelle zur Herstellung therapeutisch wirksamer VSV-LCMV-GP-Pseudotypvektoren

Migrierende VSV-LCMV-GP-Produzentenzellen werden für kr VSV-Vektoren und für replikationsinkompetente VSV-LCMV-GP-Vektoren etabliert.

### Herstellung und Charakterisierung von verschiedenen M-Varianten

Das VSV M-Protein hat zwei wesentliche Aufgaben im VSV-Zyklus: Zum einen ist es als strukturelle Komponente des Virions für den Zusammenbau und die Sprossung der Viren essentiell. Zum anderen trägt es durch seine "host shut-off" Aktivität der Wirts-Proteinsynthese und durch Induktion der Apoptose wesentlich zur viralen Pathogenese bei. Eine wichtige Funktion von M ist in diesem Zusammenhang die Hemmung des nucleozytoplasmatischen Transports von IFN mRNA. Dadurch wird der erste Abwehrmechanismus der Zelle gegen virale Infektionen unterdrückt.

Aufgrund dieser stark zytotoxischen Wirkung, ist es nicht möglich das M-Protein längerfristig in Zelllinien zu exprimieren. Es sind jedoch mehrere Mutationen im M-Protein bekannt, die zur Attenuierung des Virus führen. So konnten Irie und Mitarbeiter zeigen, dass durch Aminosäureaustausche in der sogenannten ³⁷PSAP⁴⁰ Region des M-Proteins eine VSV Mutante mit stark verringerter Zytopathogenität entstand (Irie et al., 2007). Wie ferner in Mausexperimenten gezeigt werden konnte, sind M-Varianten mit einfacher (M51R) oder mehrfachen (V221F und S226R; M33A und M51A) Mutationen außerhalb der PSAP Region ebenfalls stark attenuiert (Desforges et al., 2001; Jayakar und Whitt, 2002). Diese Viren sind nicht in der Lage, die Ausschüttung von IFN-a/β zu unterdrücken, induzieren also einen antiviralen Status, der die Tiere vor einer Infektion schützt. In Tumoren, die häufig Defekte im IFN-System aufweisen, können diese Viren sich jedoch vermehren und den Tumor lysieren. Solche attenuierten Varianten sind aus sicherheitstechnischen Gründen für die Gentherapie von besonderer Bedeutung, da sie präferentiell in Tumorzellen replizieren.

Die Erfinder haben ebenfalls eine nicht zytopathogene Variante des M-Proteins kloniert (M_{ncp}), die die genannten Mutationen M33A, M51R, V221F und S226R enthält und nicht mehr in der Lage ist, die Synthese von IFN-β zu unterdrücken. Diese Variante repliziert ähnlich effizient wie das Elternvirus auf IFN-inkompetenten Zellen, ist aber auf IFN-kompetenten Zellen attenuiert. Um eine effiziente Virusproduktion in den BM-TIC zu gewährleisten, wird diese mit einem viralen Interferonantagonisten (Haller et al., 2006) stabil transfiziert.

### Herstellung und Charakterisierung einer BM-TIC abgeleiteten Produzentenzelle für VSV-LCMV-GP-Pseudotypvektoren

Da aufgrund des gliomspezifischen Tropismus der VSV-LCMV-GP-Pseudotypen hauptsächlich Tumorzellen transduziert werden, kann alternativ auch mit M-Varianten gearbeitet werden, die noch eine gewisse Zytopathogenität zeigen. Um die toxischen Effekte in den BM-TIC gering zu halten, soll in den Zellen das antivirale MxA Protein über einen Tet-induzierbaren Promotor exprimiert werden. Retrovirale Vektoren mit Tet-induzierbarem Promotor sind bekannt (Loew et al., 2006). Das Tet-responsive Element (tTA) wird ebenfalls über einen retroviralen Vektor in die BM-TIC eingebracht. MxA ist eine interferoninduzierte GTPase mit antiviraler Aktivität gegenüber RNA Viren (Pavlovic et al., 1992; Stäheli und Pavlovic, 1991). Es liegt zytoplasmatisch in Form großer Oligomere vor, ist nicht toxisch und inhibiert über einen bislang ungeklärten Mechanismus die Replikation von VSV. LCMV-GP exprimierende BM-TIC werden mit dem Tet-regulierten MxA und dem tTa Vektor transduziert und anschließend mit den VSV-Vektoren infiziert. Solange das Antibiotikum im Medium vorhanden ist, sind die Zellen durch das MxA resistent gegenüber einer produktiven Infektion mit VSV(LCMV-GP). Erst während der *in vivo* Anwendung wird das Antibiotikum entfernt und es kommt allmählich zur viralen Replikation und zur Produktion von VSV(LCMV-GP). Da MxA mit einer Halbwertszeit von zwei Tagen sehr stabil ist, wird alternativ die Mutante MxA(L612K) verwendet. MxA(L612K) hemmt mit gleicher Effektivität die Infektion von VSV wie das Wildtypprotein, ist aber nicht in der Lage Oligomere auszubilden. Hierdurch ist es destabilisiert und verfügt über eine kurze Halbwertzeit von nur zwei Stunden (Janzen et al., 2000).

Beide Produzentenzellen, sowohl BM-TIC, die Vektoren mit nicht zytopathogenem M produzieren als auch BM-TIC, die durch MxA Expression vor VSV-bedingter Lyse geschützt sind, werden wie folgt charakterisiert:
(i) Es werden die Vektortiter und die Dauer der Vektorproduktion ermittelt.
(ii) Es wird analysiert, welchen Einfluss das Wegfrieren und Auftauen der Zellen auf den Vektortiter hat.
(iii) Es wird zunächst im Sphäroidmodell und anschließend im Tiermodell analysiert, ob die Zellen noch tumorinfiltrative Eigenschaften haben und welche Transduktionseffizienzen im Tumorgewebe erreicht werden.
(iv) Der produzierte Vektorüberstand wird auf das Vorhandensein replikationsfähiger VSV untersucht.

### Beispiel 5: Klonierung und Verpackung therapeutischer Gene

Als therapeutisches Gen gemäß der Erfindung kann ein Suizidgen benutzt werden. Beispiel dafür ist die Thymidinkinase des Herpes Simplex Virus (HSV-TK), mit der die meiste Erfahrung in der Klinik vorliegt. Durch die konstitutive Expression der HSV-TK lassen sich am Ende der Therapie Verpackungszellen und transduzierte Tumorzellen sicher eliminieren, so dass keine Gefahr von mitotisch aktiven Verpackungszellen ausgehen kann.

Zur Immuntherapie maligner Gliome, d. h. zur Verstärkung der schwach ausgeprägten lokalen Immunantwort im Gehirn, kann ein immunmodulatorisches Gen eingesetzt werden. Beispiele dafür sind Zytokine, insbesondere IL-12 und Flt3L. Nach Antigenstimulation wird IL-12 natürlicher Weise durch Antigenpräsentierende Zellen, wie dendritische Zellen, Makrophagen und B-Zellen sekretiert und vermittelt eine starke, Th1-basierte antitumorale Immunantwort. Es fördert das Wachstum und die Differenzierung naiver T-Zellen zu Th1-Zellen und wirkt somit einer gliominduzierten Suppression der T-Zellproliferation und IFNgamma Produktion entgegen. IL-12 verstärkt die zytotoxische Aktivität natürlicher Killer- und CD8+-Zellen und besitzt zudem eine anti-angiogenische Aktivität (Majewski et al., 1996). In mehreren Tiermodellen konnte bereits die Effektivität einer IL-12 vermittelten Immunantwort gegen Gliome eindrucksvoll demonstriert werden (Toda et al., 1998; Kikuchi et al., 1999). Das Zytokin Flt3L ist essentiell für die Proliferation und Differenzierung von Antigen-präsentierenden dendritischen Zellen und erscheint somit für einen Gentherapieansatz besonders gut geeignet. Sumia und Kollegen (2004) konnten nach i.c. Tranduktion von Gliomen in der Ratte/Maus mit dem Flt3L Gen einen starken lokalen Influx von dendritischen Zellen beobachten. 70% der behandelten Ratten zeigten eine Regression der Tumore und überlebten.

Die Kombination eines Zytokins mit einem Suizidgen (TK) bietet nicht nur maximale Sicherheit sondern gestattet dank des "bystander effects" eine erfolgreiche Therapie bei nur geringer Transduktionseffizienz. Dieser Ansatz vereint drei Wirkprinzipien, nämlich virale Onkolyse, Suizidgen-induzierte Apoptose und lokale Immunstimulation, um die Effizienz der Gentherapie des Glioblastoms zu erhöhen.

Zunächst werden IL-12 und Flt3L in die VSV-Vektoren kloniert. IL-12 ist speziesspezifisch aktiv, sodass für die Testung im Rattengliommodell das murine IL-12 verwendet werden muss. Humanes und murines Flt3L sind dagegen zu über 70% homolog und in beiden Spezies kreuzreaktiv. Die immunstimulatorischen Gene werden an die Position des p-Gens kloniert, während TKgfp bzw. gfp auf dem zweiten Vektor das VSV-G-Gen ersetzt. Folgende Genkombinationen werden in die beiden Vektoren des kr VSV-LCMV-GP-Pseudotypvektorsystems kloniert (Figur 5):
1) Vektor a=IL12 bzw. Flt3L + Vektor b= TKgfp (oder TK)
2) Vektor a=IL12 bzw. Flt3L + Vektor b= gfp
3) Vektor a=IL12 bzw. Flt3L + Vektor b= leer
4) Vektor a=leer + Vektor b= TKgfp (oder TK)
5) Vektor a=leer + Vektor b= gfp
6) Vektor a=leer + Vektor b= leer

Nach der Klonierung werden Gliomazellinien und BM-TIC transduziert und die Konzentration der im Überstand befindlichen Zytokine im ELISA überprüft. Werden physiologisch relevante Mengen produziert, dann erfolgt die Aufkonzentrierung der Vektoren über Ultrazentrifugation. Die Vektoren werden zunächst zur Transduktion von Sphäroiden benutzt und hier deren Fähigkeit zum "bystander killing" untersucht. Hiernach werden die Vektorüberstände zusammen mit den vorbereiteten Verpackungszellen an unseren Kooperationspartner nach Bergen geschickt. Hier erfolgt die in vivo Testung im syngenen Rattengliommodell. Es wird die Überlebensrate der behandelten Ratten nach Ganciclovirgabe evaluiert. Parallel wird die therapeutische Wirksamkeit der Immuntherapie in Abwesenheit von TK untersucht. Die Vektorkombination 6) gibt Auskunft über die Effektivität der Onkolyse. Tumorgröße und somit der Therapieerfolg werden über MRI und PET beobachtet (Miletic et al., 2007). Die Wirkung der immunmodulatorischen Gene wird anhand immunhistochemischer Methoden zum Nachweis von Infiltratzellen bewertet. Wie im folgenden Abschnitt beschrieben wird der Therapieansatz auch im Humangliommodell untersucht.

### Beispiel 6: Testung des therapeutischen Wirkprinzips in zwei Gliommodellen

Die rekombinanten VSV-Vektoren werden zunächst *in vitro* charakterisiert und anschließend im Tiermodell auf ihre Wirksamkeit untersucht. Dazu werden zwei besonders aussagekräftige Rattenmodelle eingesetzt.

Ein syngenes 9L Rattengliommodell wird zur Untersuchung immunologischer Aspekte bzw. die Durchführung immuntherapeutischer Verfahren benutzt. Dazu werden Gliomzelllinien der Ratte in die Hirne von Fisher-Ratten implantiert, die daraufhin nach einigen Tagen Tumoren entwickeln. 9L Gliosarkomzellen, die aus Fischer-Ratten isoliert wurden, dienen hierbei als Tumorzelllinien. In die so induzierten Tumoren werden dann die unterschiedlichen Vektoren bzw. Zelllinien stereotaktisch injiziert. Dieses Rattenmodel weist die folgenden Charakteristika auf:
(i) Es stellt ein gut reproduzierbares System mit einer geringen interindividuellen Varianz dar.
(ii) Die Tumoren weisen ähnliche Charakteristika wie humane Gliome auf, wie z.B. das invasive und aggressive Wachstumsverhalten sowie die Produktion von TGFβ2 als immunsuppressiven Faktor.
(iii) Das System erlaubt im Gegensatz zum SCID/Nacktmausmodell oder zum Ratten-Xenograft-Modell die Untersuchung immunologischer Aspekte bzw. die Testung immuntherapeutischer Verfahren.

Ein humanes Gliommodell in Nacktratten wird zur Testung der onkolytischen Aktivität der erfindungsgemäßen Vektoren und Zellen gegenüber humanen Gliomzellen eingesetzt. Das Ratten-Xenograft-Modell imitiert die unterschiedlichen Wachstumscharakteristika humaner Gliome. Hier wird für die Tumoretablierung keine definierte Glioblastomzelllinie, sondern primäres Tumormaterial aus Patienten verwendet, wodurch es gelingt, die Heterogenität innerhalb humaner Gliome zu imitieren (Sakariassen et al., 2006). Durch die serielle Passage von humanen Gliomen in Nacktratten können unterschiedliche Phasen der malignen Tumorentwicklung abgebildet und der Therapieerfolg untersucht werden. Das humane Gliommodell ist hervorragend zur Untersuchung von Therapieansätzen geeignet, da es folgende Charakteristika aufweist:
(i) Die Tumoren weisen ähnliche Charakteristika wie humane Gliome auf, wie z.B. das invasive und aggressive Wachstumsverhalten. Es werden keine Gliomzelllinien, sondern primäres Patientenmaterial zur Etablierung der Tumoren verwendet.
(ii) Durch die serielle Passage der Tumoren können unterschiedliche Phasen der malignen Tumorentwicklung abgebildet und der Therapieerfolg untersucht werden. Tumore der ersten Generation wachsen langsam, hoch invasiv und weisen eine geringe Neurovaskularisierung auf. Durch Passagen entwickelt sich ein stark vaskularisierter Phänotyp mit starker Proliferation und verringerter Invasion.
(iii) Der zelluläre Phänotyp hoch invasiver, nicht angiogener Gliome ähnelt dem von Tumorstammzellen. Daher ermöglicht dieses Tiermodell die Testung der therapeutischen Wirksamkeit unseres Konzepts auf die Stammzellkomponente des humanen GBM.

Zwei Kategorien von Tumoren können unterschieden werden:
(i) Frühe Tumoren mit einem langsamen, hoch invasiven Wachstumsverhalten. Diese Tumore haben nur eine geringe Gefäßneubildung.
(ii) Späte, stark durchblutete und schnell wachsende Tumoren. Dieser Phänotyp zeigt geringere Invasion.

Das humane Gliommodell eignet sich hervorragend zur Untersuchung einer Suizidgentherapie als auch zur Untersuchung der viralen Onkolyse. Es bestehen jedoch bislang keine Tiermodelle fürs humane Glioblastom, die zur Analyse der immuntherapeutischen Wirksamkeit von gentherapeutischen Verfahren herangezogen werden können.

Der Therapieerfolg wird mittels bildgebender Verfahren und schließlich mit histologischen Techniken analysiert und evaluiert. Zunächst wird die Wirkweise der sich ergänzend replizierenden VSV(LCMV-GP) Pseudotypen durch intratumorale Injektion aufkonzentrierter Vektorüberstände im Rattengliommodell untersucht. Die Transduktionseffizienz und die Verteilung der gfp-kodierenden Vektoren innerhalb der Tumore werden nach einigen Tagen anhand einer fluoreszenzmikroskopischen Analyse von Gefrierschnitten untersucht. Ferner werden Onkolyse und die Einwanderung von Immunzellen immunhistochemisch beurteilt. Zur Ergänzung wird die Wirksamkeit auch im Tiermodell für das humane Glioblastom untersucht.

Im gleichen Modell wird die Migrationsfähigkeit der BM-TIC Produzentenzellen und die Infektiosität der produzierten VSV-Vektoren untersucht. Hierfür werden die Zellen mit einem grünen Farbstoff beladen, während die freigesetzten Vektoren für rfp kodieren. Diese Vorgehensweise ermöglicht eine einfache fluoreszenzbasierte Unterscheidung zwischen BM-TIC und transduzierten Tumorzellen.

Schließlich wird die therapeutische Effizienz der einzelnen Transgen-kodierenden Pseudotypen verglichen. Dazu sollen die Verpackungszellen und parallel auch die aufkonzentrierten Vektorüberstände in etablierte 9L-Tumoren injiziert werden. Die Vektoren sollen ein immunstimulatorisches Gen in Kombination mit TK enthalten. Einige Tage nach der stereotaktischen Applikation der Vektoren bzw. der Verpackungszellen wird mit der Ganciclovirgabe begonnen. Einige der Versuchstiere erhalten Vektoren nur mit TK und dienen zur Evaluierung der therapeutischen Effizienz der immunmodulatorischen Komponente im Vektor. Nach Abschluss der Behandlung soll die Effektivität der einzelnen Therapiekonzepte anhand der Tumorgröße mit bildgebenden Verfahren (MRI; PET) bewertet werden. Ein Teil der Gehirne von immuntherapierten Ratten wird zur Charakterisierung des Immunstatus verwendet. Die Expression von MHC-Molekülen und die Verteilung von T-Zellen, Granulozyten, Makrophagen und Mikrogliazellen werden mittels immunhistochemischer Methoden an den Gefrierschnitten untersucht. Zusätzlich werden die i.c. Infiltratzellen durchflusszytometrisch hinsichtlich ihrer Zahl und phänotypischen Zusammensetzung analysiert. Die Formulierung mit dem besten therapeutischen Ergebnis wird für die Behandlung humaner Gliome adaptiert. Ein solcher Prototyp könnte dann direkt in präklinische Sicherheitsuntersuchungen, und nach der Etablierten der entsprechenden Verfahren zu GMP-Produktion, in die klinische Testung überführt werden.

### Literatur

1. Barber GN. Vesicular stomatitis virus as an oncolytic vector. Viral Immunol. 2004; 17(4):516-27
2. Beyer WR, Westphal M, Ostertag W, von Laer D. Oncoretrovirus and lentivirus vectors pseudotyped with lymphocytic choriomeningitis virus glycoprotein: generation, concentration, and broad host range. J Virol. 2002; 76:1488-95
3. Carlsson J, Yuhas JM. Liquid-overlay culture of cellular spheroids. Recent Results Cancer Res. 1984; 95:1-23
4. Culver KW, Ram Z, Wallbridge S, Ishii H, Oldfield EH, Blaese RM. In vivo gene transfer with retroviral vectorproducer cells for treatment of experimental brain tumors. Science. 1992 Jun 12; 256(5063):1550-2
5. Cutter JL, Kurozumi K, Chiocca EA, Kaur B. Gene therapeutics: the future of brain tumor therapy? Expert Review of Anticancer Therapy. 2006; 6(7):1053-1064
6. Dai C, Holland EC. Glioma models. Biochim Biophys Acta. 2001 Aug 31; 1551(1):M19-27
7. Desforges M, Charron J, Berard S, Beausoleil S, Stojdl DF, Despars G, Laverdiere B, Bell JC, Talbot PJ, Stanners CP, Poliquin L. Different host-cell shutoff strategies related to the matrix protein lead to persistence of vesicular stomatitis virus mutants on fibroblast cells. Virus Res. 2001 Jul; 76(1):87-102
8. Finke S, Conzelmann KK. Recombinant rhabdoviruses: vectors for vaccine development and gene therapy. Curr Top Microbiol Immunol. 2005; 292:165-200
9. Fischer YH, Miletic H, Giroglou T, Litwak S, Stenzel W, Neumann H, von Laer D. A retroviral packaging cell line for pseudotype vectors based on glioma-infiltrating progenitor cells. Journal of Gene Medicine. 2007; 9:335-44
10. Fukuhara H, Martuza RL, Rabkin SD, Ito Y, Todo T. Oncolytic herpes simplex virus vector g47delta in combination with androgen ablation for the treatment of human prostate adenocarcinoma. Clin Cancer Res. 2005 Nov 1; 11(21):7886-90
11. Garbutt M, Liebscher R, Wahl-Jensen V, Jones S, Moller P, Wagner R, Volchkov V, Klenk HD, Feldmann H, Stroher U. Properties of replication-competent vesicular stomatitis virus vectors expressing glycoproteins of filoviruses and arenaviruses. J Virol. 2004 May; 78(10):5458-65
12. Ge J, Wen Z, Wang X, Hu S, Liu Y, Kong X, Chen H, Bu Z. Generating vesicular stomatitis virus pseudotype bearing the severe acute respiratory syndrome coronavirus spike envelope glycoprotein for rapid and safe neutralization test or cell-entry assay. Ann NY Acad Sci. 2006 Oct; 1081:246-8
13. Irie T, Carnero E, Okumura A, Garcia-Sastre A, Harty RN. Modifications of the PSAP region of the matrix protein lead to attenuation of vesicular stomatitis virus in vitro and in vivo. J Gen Virol. 2007 Sep; 88(Pt 9):2559-67
14. Hanika A, Larisch B, Steinmann E, Schwegmann-Wessels C, Herrler G, Zimmer G. Use of influenza C virus glycoprotein HEF for generation of vesicular stomatitis virus pseudotypes. J Gen Virol. 2005 May; 86(Pt 5):1455-65
15. Haller O, Kochs G, Weber F. The interferon response circuit: induction and suppression by pathogenic viruses. Virology. 2006 Jan 5; 344(1):119-30
16. Holland EC. Gliomagenesis: genetic alterations and mouse models. Nat Rev Genet. 2001 Feb; 2(2):120-9
17. Janzen C, Kochs G, Haller O. A Monomeric GTPase-Negative MxA Mutant with Antiviral Activity. J Virol. 2000 Sept.; 74(17):8202-6
18. Jayakar HR, Whitt MA. Identification of two additional translation products from the matrix (M) gene that contribute to vesicular stomatitis virus cytopathology. J Virol. 2002 Aug; 76(16):8011-8
19. Jiang Y, Jahagirdar BN, Reinhardt RL, Schwartz RE, Keene CD, Ortiz-Gonzalez XR, Reyes M, Lenvik T, Lund T, Blackstad M, Du J, Aldrich S, Lisberg A, Low WC, Largaespada DA, Verfaillie CM. Pluripotency of mesenchymal stem cells derived from adult marrow. Nature. 2002; 418:41-9
20. Kikuchi, T., Joki, T., Akasaki, Y., Abe, T, Ohno, T. Cancer Lett. 1999; 135:47-51
21. Kleihues, P. and Cavenee, W.K. World Health Organization Classification of Tumours- Pathology and Genetics, Tumours of the Nervous system. 2000 (International Agency for Research on cancer, Lyon)
22. Loew R, Vigna E, Lindemann D, Naldini L, Bujard H. Retroviral vectors containing Tet-controlled bidirectional transcription units for simultaneous regulation of two gene activities. J Mol Gen Med. 2006; 2(1):107-118
23. Lun X, Senger DL, Alain T, Oprea A, Parato K, Stojdl D, Lichty B, Power A, Johnston RN, Hamilton M, Parney I, Bell JC, Forsyth PA. Effects of intravenously administered recombinant vesicular stomatitis virus (VSV(deltaM51)) on multifocal and invasive gliomas. J Natl Cancer Inst. 2006 Nov 1; 98(21):1546-57
24. Majewski, S., Marczak, M., Szmurlo, A., Jablonska, S. & Bollag, W. J. Interleukin-12 inhibits angiogenesis induced by tumor cell lines in vitro. Invest. Dermatol. 1996; 106:1114-8
25. Miletic H, Bruns M, Tsiakas K, Vogt B, Rezai R, Baum C, Kuhlke K, Cosset FL, Ostertag W, Lother H, von Laer D. Retroviral vectors pseudotyped with lymphocytic choriomeningitis virus. J. Virol. 1999; 73:6114-6
26. Miletic H, Fischer YH, Neumann H, Hans V, Stenzel W, Giroglou T, Hermann M, Deckert M, Von Laer D. (2004). Selective transduction of malignant glioma by lentiviral vectors pseudotyped with lymphocytic choriomeningitis virus glycoproteins. Hum Gene Ther.15: 1091-100
27. Miletic H, Fischer YH, Litwak S, Giroglou T, Waerzeggers Y, Winkeler A, Li H, Himmelreich U, Lange C, Stenzel W, Deckert M, Neumann H, Jacobs AH, von Laer D. Bystander Killing of Malignant Glioma by Bone Marrow-Derived Tumor Infiltrating Progenitor Cells Expressing a Suicide Gene. Molecular Therapy 2007; 15:1373-81
28. Miletic H, Fischer YH, Giroglou T; Rueger MA, Winkeler A, Li H, Himmelreich U, Stenzel W, Jacobs AH, von Laer D. Normal brain cells contribute to the bystander effect in suicide gene therapy of malignant glioma. Jorurnal of Clinical Cancer Research, 2007b; in press
29. Owens RJ, Rose JK. Cytoplasmic domain requirement for incorporation of a foreign envelope protein into vesicular stomatitis virus. J Virol. 1993 Jan; 67(1):360-5
30. Pavlovic J, Haller O, Staeheli P. Human and mouse Mx proteins inhibit different steps of the influenza virus multiplication cycle. J Virol. 1992 Apr; 66(4):2564-9
31. Riepl C, Mandl C. Two non-infectious flaviviruses can form an infectious alliance. Nürnberg, 2007. Poster at the annual Meeiting of the Gesellschaft für Virologie
32. Sakariassen PO, Prestegarden L, Wang J, Skaftnesmo KO, Mahesparan R, Molthoff C, Sminia P, Sundlisaeter E, Misra A, Tysnes BB, Chekenya M, Peters H, Lende G, Kalland KH, Oyan AM, Petersen K, Jonassen I, van der Kogel A, Feuerstein BG, Terzis AJ, Bjerkvig R, Enger PO. Angiogenesis-independent tumor growth mediated by stemlike cancer cells. Proc Natl Acad Sci U S A. 2006 Oct 31; 103(44):16466-71
33. Shinosaki K, Ebert O, Suriawinata A, Thung SN, Woo SL. Prophylactic alpha interferon treatment increases the therapeutic index of oncolytic vesicular stomatitis virus virotherapy for advanced hepatocellular carcinoma in immune-competent rats. J Virol. 2005 Nov; 79(21):13705-13
34. Shir A, Levitzki A. Gene therapy for glioblastoma: future perspective for delivery systems and molecular targets. Cell Mol Neubiol. 2001 Dec; 21:645-56
35. Short MP, Choi BC, Lee JK, Malick A, Breakefield XO, Martuza RL. Gene delivery to glioma cells in rat brain by grafting of a retrovirus packaging cell line. J Neurosci Res. 1990 Nov; 27(3):427-39
36. Staeheli P, Pavlovic J. Inhibition of vesicular stomatitis virus mRNA synthesis by human MxA protein. J Virol. 1991 Aug; 65(8):4498-501
37. Sumia A, Curtin JF, Zirger JM, Xiong W, King GD, Barcia C, Liu C, Puntel1 M, Goverdhana S, Lowenstein PR, Castro MG. Inflammatory and Anti-glioma Effects of an Adenovirus Expressing Human Soluble Fms-like Tyrosine Kinase 3 Ligand (hsFlt3L): Treatment with hsFlt3L Inhibits Intracranial Glioma Progression. Molecular Therapy. 2004; 10:1071-84
38. Tani H, Komoda Y, Matsuo E, Suzuki K, Hamamoto I, Yamashita T, Moriishi K, Fujiyama K, Kanto T, Hayashi N, Owsianka A, Patel AH, Whitt MA, Matsuura Y. Replication-competent recombinant vesicular stomatitis virus encoding hepatitis C virus envelope proteins. J Virol. 2007 Aug; 81(16):8601-12. Epub 2007 Jun 6
39. Toda, M., Martuza, R. L., Kojima, H. & Rabkin, S. D. In situ cancer vaccination: an IL-12 defective vector/replication-competent herpes simplex virus combination induces local and systemic antitumor activity. J. Immunol. 1998; 160:4457-4464
40. Trajcevski S, Solly SK, Frisen C, Trenado A, Cosset FL, Klatzmann D. Characterization of a semi-replicative gene delivery system allowing propagation of complementary defective retroviral vectors. J Gene Med. 2005 Mar; 7(3):276-87
41. Tyminski E, Leroy S, Terada K, Finkelstein DM, Hyatt JL, Danks MK, Potter PM, Saeki Y, Chiocca EA. Brain tumor oncolysis with replication-conditional herpes simplex virus type 1 expressing the prodrug-activating genes, CYP2B1 and secreted human intestinal carboxylesterase, in combination with cyclophosphamide and irinotecan. Cancer Res. 2005 Aug 1; 65(15):6850-7
42. Wollmann G, Robek MD, van den Pol AN. Variable deficiencies in the interferon response enhance susceptibility to vesicular stomatitis virus oncolytic actions in glioblastoma cells but not in normal human glial cells. J Virol. 2007 Feb; 81(3):1479-91
43. Zimmer G, Conzelmann KK, Herrler G. Cleavage at the furin consensus sequence RAR/KR(109) and presence of the intervening peptide of the respiratory syncytial virus fusion protein are dispensable for virus replication in cell culture. J Virol. 2002 Sep; 76(18):9218-24

## Patentansprüche

1. VSV-LCMV-GP-Pseudotypvektor, **dadurch gekennzeichnet, dass** er ein für ein Glykoprotein GP des lymphozytären Choriomeningitisvirus (LCMV) kodierendes Gen und kein funktionsfähiges Gen für Hüllprotein G des VSV enthält.

2. Der VSV-LCMV-GP-Pseudotypvektor nach Anspruch 1, **dadurch gekennzeichnet, dass** das Hüllprotein G des VSV durch GP des LCMV ersetzt ist.

3. Der VSV-LCMV-GP-Pseudotypvektor nach Anspruch 1 oder 2, **dadurch gekennzeichnet, dass** er mindestens ein Transgen enthält, welches insbesondere für ein Suizidprotein, ein immunstimulatorisches Protein oder ein Markerprotein kodiert.

4. VSV-LCMV-GP-Pseudotypvektorsystem, **dadurch gekennzeichnet, dass** es mindestens zwei komplementierend replizierende VSV-Vektoren umfasst, wobei es für N-, L-P- und M-Protein des VSV kodierende Gene *n, l, p* und *m*, ein für ein Glykoprotein GP des LCMV kodierendes Gen *gp* und kein funktionsfähiges für Hüllprotein G des VSV kodierendes Gen enthält und wobei auf jedem Vektor des Systems eins der Gene *n*, *l, p, m* und *gp* fehlt und wobei das fehlende Gen auf einem anderen Vektor des Vektorsystems vorhanden ist.

5. Das VSV-LCMV-GP-Pseudotypvektorsystem nach Anspruch 4, **dadurch gekennzeichnet, dass** es mindestens ein Transgen enthält, welches insbesondere für ein Suizidprotein, ein immunstimulatorisches Protein oder ein Markerprotein kodiert.

6. Pseudotypisiertes VSV-Virion, **dadurch gekennzeichnet, dass** es ein GP-Protein des LCMV als Hüllprotein enthält.

7. Virusproduzentenzelle, **dadurch gekennzeichnet, dass** sie ein pseudotypisiertes VSV-Virion nach Anspruch 6 produziert.

8. Die Virusproduzentenzelle nach Anspruch 7, **dadurch gekennzeichnet, dass** sie eine adulte Stammzelle, insbesondre eine multipotente adulte Progenitorzelle (MAPC), eine neuronale Stammzelle (NSC), eine mesenchymale Stammzelle (MSC) oder eine BM-TIC Zelle (bone *m*arrow derived tumor infiltrating cell), ist.

9. Die Virusproduzentenzelle nach Anspruch 7 oder 8, **dadurch gekennzeichnet, dass** sie eine oder mehrere Expressionskassetten für die Expression mindestens eines der Gene aus der Gruppe bestehend aus für N-, L-, P- und M-Proteine des VSV kodierenden Genen *n*, *l, p* und *m* sowie einem für ein Glykoprotein GP des LCMV kodierenden Gen *gp* enthält.

10. Die Virusproduzentenzelle nach einem der Ansprüche 7 bis 9, **dadurch gekennzeichnet, dass** sie einen Gentransfervektor zur Verpackung in das pseudotypisierte VSV-Virion enthält, wobei der Gentransfervektor ein Transgen enthält, welches insbesondere für ein Suizidprotein, ein immunstimulatorisches Protein oder ein Markerprotein kodiert.

11. Verfahren zum Transfer eines Transgens in eine Zelle *in vitro*, **dadurch gekennzeichnet, dass** man die Zelle mit einem pseudotypisierten VSV-Virion, nach Anspruch 6 transduziert, wobei das Virion ein Transgen enthält.

12. Verfahren zum Transfer eines Transgens in eine Zelle *in vitro*, **dadurch gekennzeichnet, dass** man die Zelle mit einer Virusproduzentenzelle nach Anspruch 10 kontaktiert.

13. Der VSV-LCMV-GP-Pseudotypvektor nach einem der Ansprüche 1 bis 3 zur Verwendung zur Therapie eines soliden Tumors, insbesondere eines Hirntumors.

14. Das VSV-LCMV-GP-Pseudotypvektorsystem nach Anspruch 4 oder 5 zur Verwendung zur Therapie eines soliden Tumors, insbesondere eines Hirntumors.

15. Die Virusproduzentenzelle nach einem der Ansprüche 7 bis 10 zur Verwendung zur Therapie eines soliden Tumors, insbesondere eines Hirntumors.

16. Pharmazeutische Zusammensetzung, **dadurch gekennzeichnet, dass** sie einen VSV-LCMV-GP-Pseudotypvektor nach einem der Ansprüche 1 bis 3, ein VSV-LCMV-GP-Pseudotypvektorsystem nach Anspruch 4 oder 5, ein pseudotypisiertes VSV-Virion nach Anspruch 6 oder eine Virusproduzentenzelle nach einem der Ansprüche 7 bis 10 enthält.

## Claims

1. VSV-LCMV-GP pseudotype vector, **characterized in that** the vector comprises a gene coding for a glycoprotein GP of lymphocyte choriomeningitis virus (LCMV) and lacks a functional gene coding for envelope protein G of VSV.

2. The VSV-LCMV-GP pseudotype vector of claim 1, **characterized in that** the envelope protein G of VSV is replaced by the glycoprotein GP of LCMV.

3. The VSV-LCMV-GP pseudotype vector of claim 1 or 2, **characterized in that** the vector comprises at least one transgene, which preferably encodes a suicide protein, an immunostimulatory protein, or a marker protein.

4. VSV-LCMV-GP pseudotype vector system, **characterized in that** the system comprises at least two complementary replicating VSV vectors, wherein the system comprises genes *n*, *l, p* and *m* coding for proteins N, L, P and M of VSV, a gene gp coding for a glycoprotein GP of LCMV and lacks a functional gene coding for envelope protein G of VSV, wherein each vector of the system lacks one of the genes *n, l, p*, *m* and *gp*, and wherein the lacking gene is present on any other vector of the system.

5. The VSV-LCMV-GP pseudotype vector system of claim 4, **characterized in that** the system comprises at least one transgene, which preferably encodes a suicide protein, an immunostimulatory protein, or a marker protein.

6. Pseudotyped VSV virion, **characterized in that** the virion comprises a glycoprotein GP of LCMV as envelope protein.

7. Virus producing cell, **characterized in that** the cell produces a pseudotyped VSV virion of claim 6.

8. The virus producing cell of claim 7, **characterized in that** the cell is an adult stem cell, which is preferably a multipotent adult progenitor cell (MAPC), a neural stem cell (NSC), a mesenchymal stem cell (MSC) or a bone marrow derived tumor infiltrating cell (BM-TIC).

9. The virus producing cell of claim 7 or 8, **characterized in that** the cell comprises one or more expression cassettes for the expression of at least one of the genes selected from the group consisting of genes *n*, *l*, *p* and *m* coding for proteins N, L, P and M of VSV and a gene gp coding for glycoprotein GP of LCMV.

10. The virus producing cell of any one of claims 7 to 9, **characterized in that** the cell comprises a gene transfer vector for packaging into the pseudotyped VSV virion, wherein the gene transfer vector comprises a transgene, which preferably encodes a suicide protein, an immunostimulatory protein and/or a marker protein.

11. Method for transfer of a transgene into a cell *in vitro*, **characterized in that** the cell is transduced with a pseudotyped VSV virion of claim 6, wherein the virion comprises a transgene.

12. Method for transfer of a transgene into a cell *in vitro,* **characterized in that** the cell is contacted with a virus producing cell of claim 10.

13. The VSV-LCMV-GP pseudotype vector of any one of claims 1 to 3 for use in the therapy of a solid tumor, preferably a brain tumor.

14. The VSV-LCMV-GP pseudotype vector system of claim 4 or 5 for use in the therapy of a solid tumor, preferably a brain tumor.

15. The virus producing cell of any one of claims 7 to 10 for use in the therapy of a solid tumor, preferably a brain tumor.

16. Pharmaceutical composition, **characterized in that** the composition comprises a VSV-LCMV-GP pseudotype vector of any one of claims 1 to 3, a VSV-LCMV-GP pseudotype vector system of claim 4 or 5, a pseudotyped VSV virion of claim 6, or a virus producing cell of any one of claims 7 to 10.

## Revendications

1. Vecteur pseudotypé VSV-LCMV-GP, **caractérisé en ce que** le vecteur comprend un gène codant une glycoprotéine GP du virus de la chorioméningite lymphocytaire (MCMV) et est dépourvu d'un gène fonctionnel codant la protéine d'enveloppe G de VSV.

2. Vecteur pseudotypé VSV-LCMV-GP selon la revendication 1, **caractérisé en ce que** la protéine d'enveloppe G de VSV est remplacée par la glycoprotéine GP de LCMV.

3. Vecteur pseudotypé VSV-LCMV-GP selon la revendication 1 ou 2, **caractérisé en ce que** le vecteur comprend au moins un transgène, qui de préférence code une protéine suicide, une protéine immunostimulatrice, ou une protéine de marquage.

4. Système de vecteur pseudotypé VSV-LCMV-GP, **caractérisé en ce que** le vecteur comprend au moins deux vecteurs VSV réplicables complémentaires, le système comprenant les gènes *n, l*, *p* et *m* codant les protéines N, L, P et M de VSV, un gène *gp* codant une glycoprotéine GP de LCMV et étant dépourvu d'un gène fonctionnel codant la protéine d'enveloppe G de VSV, chaque vecteur du système étant dépourvu de l'un des gènes *n, l*, *p, m* et *gp,* et le gène manquant étant présent sur un autre vecteur quelconque du système.

5. Système de vecteur pseudotypé VSV-LCMV-GP selon la revendication 4, **caractérisé en ce que** le système comprend au moins un transgène, qui de préférence code une protéine suicide, une protéine immunostimulatrice, ou une protéine de marquage.

6. Virion VSV pseudotypé, **caractérisé en ce que** le virion comprend une glycoprotéine GP de LCMV en tant que protéine d'enveloppe.

7. Cellule productrice de virus, **caractérisée en ce que** la cellule produit un virus VSV pseudotypé selon la revendication 6.

8. Cellule productrice de virus selon la revendication 7, **caractérisée en ce que** la cellule est une cellule souche adulte, qui est de préférence une cellule progénitrice multipotente adulte (MAPC), une cellule souche neurale (NSC) ou une cellule infiltrante tumorale issue de moelle osseuse (BM-TIC).

9. Cellule productrice de virus selon la revendication 7 ou 8, **caractérisée en ce que** la cellule comprend une ou plusieurs cassettes d'expression pour l'expression d'au moins l'un des gènes choisis dans le groupe constitué par les gènes *n*, *l*, *p* et *m* codant les protéines N, L, P et M de VSV et un gène *gp* codant une glycoprotéine GP de LCMV.

10. Cellule productrice de virus selon l'une quelconque des revendications 7 à 9, **caractérisée en ce que** la cellule comprend un vecteur de transfert de gène pour l'encapsidation dans le virion VSV pseudotypé, le vecteur de transfert de gène comprenant un transgène, qui de préférence code une protéine suicide, une protéine immunostimulatrice, et/ou une protéine de marquage.

11. Procédé pour le transfert d'un transgène dans une cellule in vitro, **caractérisé en ce que** la cellule est transduite avec un virion VSV pseudotypé selon la revendication 6, le virion comprenant un transgène.

12. Procédé pour le transfert d'un transgène dans une cellule in vitro, **caractérisé en ce qu'**on met la cellule en contact avec une cellule productrice de virus selon la revendication 10.

13. Vecteur pseudotypé VSV-LCMV-GP selon l'une quelconque des revendications 1 à 3, pour utilisation dans la thérapie d'une tumeur solide, de préférence une tumeur du cerveau.

14. Système de vecteur pseudotypé VSV-LCMV-GP selon la revendication 4 ou 5, pour utilisation dans la thérapie d'une tumeur solide, de préférence une tumeur du cerveau.

15. Cellule productrice de virus selon l'une quelconque des revendications 7 à 10, pour utilisation dans la thérapie d'une tumeur solide, de préférence une tumeur du cerveau.

16. Composition pharmaceutique, **caractérisée en ce que** la composition comprend un vecteur pseudotypé VSV-LCMV-GP selon l'une quelconque des revendications 1 à 3, un système de vecteur pseudotypé VSV-LCMV-GP selon la revendication 4 ou 5, un virion VSV pseudotypé selon la revendication 6, ou une cellule productrice de virus selon l'une quelconque des revendications 7 à 10.
